(19) Europäisches Patentamt
European Patent Office
Office européen des brevets

(11) Publication number: 0 127 396
A2

# (12) EUROPEAN PATENT APPLICATION

(21) Application number: 84303354.9

(22) Date of filing: 17.05.84

(51) Int. Cl.[3]: C 07 C 85/06
C 07 C 87/64, B 01 J 21/04
B 01 J 27/06

(30) Priority: 20.05.83 US 496369

(43) Date of publication of application:
05.12.84 Bulletin 84/49

(84) Designated Contracting States:
BE DE FR GB IT NL

(71) Applicant: THE HALCON SD GROUP, INC.
2 Park Avenue
New York, N.Y. 10016(US)

(72) Inventor: Becker, Mitchell
778 Downing Street
Teaneck New Jersey 07666(US)

(72) Inventor: Sachs, Howard M.
3840 Greystone Avenue
Riverdale New York 10463(US)

(74) Representative: Cropp, John Anthony David et al,
MATHYS & SQUIRE 10 Fleet Street
London, EC4Y 1AY(GB)

## (54) Process and catalyst for the production of aromatic amines.

(57) A process for the amination of phenolic compounds, such as phenol, by contacting them with an aromatic amine, such as aniline, in the presence of a solid heterogeneous, low alkali, acidic alumina catalyst at elevated temperatures and superatmospheric pressures, to produce compounds such as diphenylamine. An improved process of catalyst preparation is also disclosed.



Croydon Printing Company Ltd

## BACKGROUND OF THE INVENTION

### Field of the Invention

This invention is related to a process and an accompanying catalyst for the production of aromatic amines from the reaction of selected phenols with certain aromatic amines and to an improved acidic alumina catalyst particularly useful in this reaction; more particularly, the invention is related to the preparation of diphenylamine (DPA) from phenol and aniline using a solid, heterogeneous acidic, fluorided alumina catalyst.

### Description of the Prior Art

DPA is a well known industrial chemical having a variety of usages, such as in the production of antioxidants for elastomers and in the manufacture of azo dyes, and can be prepared by a variety of methods. Generally, DPA has been prepared commercially by catalytically deammoniating or self-condensing aniline. British Patent No. 752,859 discloses such a process in which aniline is reacted in the presence of activated alumina or titania. U.S. Patent 3,071,619 discloses a liquid-phase process involving the use of $NH_4BF_4$ as the catalyst. A 1950 publication, H. Hoelscher et al., Ind. Eng. Chem. (Vol. 42(8) pp. 1558-62 discloses that aniline can be contacted and reacted in the presence of activated alumina above 400°C to produce DPA. The catalyst, however, loses a significant amount of activity after only 10 hours and must be regenerated.

Processes for the production of a variety of aniline based compounds from the liquid or the vapor-phase amination of phenolic based compounds with ammonia have also been described in the literature. Generally, these reactions occur under conditions of elevated pressures and temperatures in the presence of catalysts containing silica and alumina. One such aniline reaction process is disclosed in U.S. Patent No. 3,272,365, wherein phenol and ammonia are combined over a silica-alumina catalyst at temperatures between 400-480°C. U.S. Patent 3,860,650

describes a process for the production of organic amines by the amination of phenolic type compounds, using a catalyst consisting essentially of an amorphous alumina gel containing less than 1.0 wt. % of alkali metal.

A 1968 publication, J. Peri, J. of Phys. Chem., pp. 2917-25, discloses that infrared studies of absorbed $CO_2$ and CO indicate that fluoriding strengthens certain strong Lewis acid sites ($\alpha$ sites) on $\gamma$ - alumina.

It had been proposed to produce DPA and other similar compounds from the catalytic reaction of aniline with phenol or other phenolic compounds, viz;

$$C_6H_5NH_2 + C_6H_5OH \text{ —— } C_6H_5NHC_6H_5 + H_2O$$

Such a process is described in U.S. Patent No. 2,824,137 which employs a titanium catalyst; in British Patent 1,541,153 which uses phosphoric acid as a catalyst, and in Japanese Patent Publication 75/07,061, which carries out the reaction in the presence of alumina. British Patent 1,402,707 describes a process for the production of DPA comprising the passing of aniline over an alumina catalyst treated with $BF_3$. U.S. Patent 3,944,613 discloses a liquid-phase process for the production of DPA which features a silica/alumina catalyst. GB-A-2128610 ———— discloses a process for the production of variable amounts of aniline and DPA using a feedstock of ammonia and phenol by a method of integrating a two reactor operation. The processes revealed by these references, however, exhibit relatively low conversions and selectivities and their accompanying catalysts tend to have very short lifetimes. It had long been recognized that while phenolic-based compounds frequently will easily react with aniline, the reaction of aniline with phenol itself is far more difficult.

Catalysts useful in the commercial production of aromatic amines such as DPA must satisfy several performance requirements if they are to be economical. Satisfactory catalytic activity, often expressed in terms of the percentage of raw material conversion to the

desired product under a particular process environment, is a major concern. The more active the catalyst the smaller will be the reaction space, or volume, required for a particular rate of production. Alternatively, a much lower reaction temperature or reactant flow rate is required to produce a desired amount of product if an active catalyst is available.

Accordingly it is an object of this invention to provide a suitable catalyst for the amination of phenolic compounds in either a liquid or a vapor phase reaction, which has sufficient activity at relatively low temperatures and within a large temperature range, and which is particularly selective to the amination reaction.

It is a further object of this invention to provide a catalyst and a method of preparation thereof, which results in the catalyst having a much longer activity period than currently available catalysts.

It is another object of this invention to provide a process and accompanying catalyst for the preparation of diphenylamine and similar compounds by the amination of phenol and other phenolic based compounds so as to produce the desired product with attractive conversions and high selectivities, and yet which avoids the drawbacks and disadvantages of presently available processes for the production of diphenylamine.

SUMMARY

These and other objects of the invention have been attained through the development of a novel process for the amination of phenolic compounds, such as phenol, by contacting the phenolic compound with an aromatic amine, such as aniline, in the presence of a solid, heterogeneous, acidic alumina catalyst, e.g., a low alkali, acidic, halide-treated alumina, so as to enable the resulting catalytic reaction to display sufficient activity over a substantially wider and lower range of operating temperatures, e.g., about 330-385°C, in the reaction of phenol with aniline to form DPA, than is currently possible.

The invention further comprises the formation of a low alkali, e.g., at most about 0.2 wt. % of alkali metal, high acidic, heterogeneous, alumina catalyst having a surface area of at least about 150 $M^2/g$, and a halide, most preferably fluoride, content of between 2-15 wt. %. The catalyst is preferably prepared by first being wetted in a steam bath for an effective time, then, if the alkali content is high, acid treating the catalyst by contacting it with an aqueous acidic solution, such as acetic acid, to reduce the alkali metal content to below the desired level. The catalyst is preferably further acidified by contacting it with an aqueous solution of fluoride ion, i.e., ammonium bifluoride, $NH_4FHF$. The treated catalyst is washed and dried at low temperatures, and does not require a high temperature calcination before usage.

## DETAILED DESCRIPTION

### A. The Class of Reactions

Suitable phenolic based compounds which can be reacted with an aromatic amine include the monohydric mono and polynuclear phenols, such as phenol, chlorophenol, pyrocatechol, ortho-, meta-, and paraxylenols, hydroquinone, orthocresol, metacresol, paracresol, alpha and beta naphthols, indanols having at least one -OH group on the aromatic portion of the molecule, resorcinol, phloroglucinol, 4,4-dihydroxybiphenyl, mesitol, thymol, carvacrol, guaiacol, o-eugenol, orcinol, pyrogallol, hydroxyanthracene, 2-hydroxyanthracene and the like. As illustrated by the aforementioned compounds, the phenols can be any mono or polyhydric or mono or polynuclear phenol.

Aromatic amines that will react with aromatic phenols in accordance with this invention include mono-polyaromatic primary amines and substituted derivatives thereof, such as aniline, the toluidines, the xylidenes, alphanaphthyl amine, beta-naphthyl amine, p-phenylene diamine, p-tolylene diamine, p-amino diphenyl amine, benzidine, tolylene diamines, xylylene diamines and the like.

The process, in the broadest embodiment, comprises contacting a phenolic compound, such as phenol, with an aromatic amine, such as aniline, in the presence of a solid, heterogeneous, acidic, alumina catalyst; the catalyst having sufficient activity to produce acceptable reaction rates at lower operating temperatures, thus also creating a substantially greater operable temperature range, and also substantially increasing the catalyst cycle life before deactivation occurs. The preferred alumina catalyst in the amination process is prepared by acid treating and fluoriding operations which increase the acidity and lower the alkali metal content to at most 0.2 wt. %.

The range of acceptable concentrations of the aromatic amine in relation to the phenolic compound may, in the broadest embodiment, vary from about 0.5:1 to 2:1 moles of amine per mole of phenolic compound.

The reaction between the phenolic compound and the aromatic amine may be carried out in the vapor phase, i.e., using a narrower embodiment in which phenol and aniline are the representative examples, the phenol and the aniline are prevaporized and passed through a fixed bed of the catalyst while under appropriate pressure and temperature conditions selected to maintain vapor phase conditioning. Alternatively, the reaction can be carried out in the liquid phase, wherein liquid phenol and liquid aniline are brought into contact with the alumina catalyst.

The liquid-phase reaction is preferred, and in such a system the catalyst may be mixed with the reactants in a batch-type process, or, a stream of phenol and aniline may be passed continuously through a bed of the alumina catalyst. The liquid-phase embodiments are, of course, carried out under appropriate pressure conditions which maintain the liquid phase.

As can be seen from the following equation, viz;

$$C_6H_5OH + C_6H_5NH_2 \longrightarrow C_6H_5NH\,C_6H_5 + H_2O;$$

The reaction between phenol and aniline to produce one mole of diphenylamine stoichiometrically requires one mole of aniline for each mole of phenol, and the process of this invention can be carried out with the two reactants in that proportion. However, either the phenol or the aniline can be in molar excess but ordinarily should not exceed a 2:1 excess of aniline or a 2:1 excess of phenol.

A surprising and valuable result of the process is its ability to display and maintain an acceptable activity at temperature ranges well below current state of the art operating temperature ranges for the particular class of reactions in question. For example, using the cited DPA reaction, this process can now be effectively operated with sufficient activity on an industrial scale within a temperature range of about 330-385°C, whereas the prior art was unable to operate effectively below about 360°C, resulting in a much smaller range of effective catalytic activity, since above 385°C the reaction is no longer sufficiently selective to the desired product. Since catalyst activity gradually lessens during operation, the process temperature is correspondingly increased during the operation in order to generate an effective amount of catalytic activity. Consequently, it is readily apparent that the ability to reduce the starting process temperature will lead to a much longer process and catalyst life, and such considerations frequently are the critical parameter in industrial catalytical processes. In the broadest embodiment, this particular reaction will undergo conversions at temperatures ranging from about 300 to 450°C, although as mentioned earlier, lower temperatures tend to reduce the reaction rate to unacceptable levels and higher temperatures are accompanied by significantly greater byproduct formation.

As previously mentioned, when the process is carried out in the preferred liquid-phase system, adequate pressure must be applied to maintain this state. Typically, pressures of 600 to 1000 psia are employed, but it is to be

emphasized that pressure is not a critical parameter of the process. For vapor-phase operation, typical pressures lie in the range from atmospheric to 400 psia.

When the liquid-phase process is carried out in a batch system, the phenol, aniline and the preferred alumina catalyst are charged to an appropriate vessel capable of withstanding the applied pressure, such as an autoclave, and the liquid mixture is stirred and heated at the reaction temperature for a predetermined length of time. The amount of catalyst present is suitably in the range of about 1 to 20 wt. % of the charge, and preferably about 3 to 10 wt. %. Ordinarily, reaction times of 1 to 20 hours are suitable, greater amounts of diphenylamine being generally produced with greater reaction times. Increased catalyst concentrations of course, produce greater quantities of product per unit time.

When the liquid-phase reaction is carried out in a fixed bed system, a liquid mixture of phenol and aniline is continuously passed through a bed of the alumina catalyst which is contained in a suitable reaction column or tube. Ordinarily, the stream is passed upwardly through the bed. The rate of feeding the phenol-aniline stream through the bed can vary over a wide range, with the rate being selected to maintain a liquid hourly space velocity ranging between 0.01 to 0.50 $hr^{-1}$. Liquid hourly space velocity is in this case determined by dividing the volume of the phenol-aniline mixture per hour by the volume of catalyst in the bed, e.g., a feed rate of phenol-aniline mixture in liters per hour and the catalyst volume in liters. In the case of liquid phase batch operation, the reaction product mixture is first filtered to remove the solid catalyst and is then treated to separate the evolved water and to recover the product diphenylamine from the unreacted aniline and phenol. When a fixed bed catalyst system is used, there is, of course, no filtration step to remove the catalyst and the liquid effluent is ready for treatment to separate its components. The recovered

aniline and phenol feedstock can then be used for a further batch reaction, or in the case of a continuous system, can be directly recycled to the reactor for further contact with the catalyst bed. In the case of vapor-phase reaction, the reaction product stream is first condensed and is then treated in the manner described for the liquid-phase reaction stream.

By following the process of this invention, selectivities of the order of about 90 to 97 are readily achieved along with significant conversions of the reactants. As mentioned, catalyst concentration and reaction or contact time will influence conversions and thus conversions can be varied by varying these two parameters to the extent desired.

B. The Catalyst

The preferred catalyst for this invention before activation consists essentially of a solid, heterogeneous, acidic alumina having a substantially crystalline microstructure. By "consisting essentially of" is meant a catalyst containing, on a water free basis, at least 90% by wt. of alumina and, preferably, at least 95% of alumina. The catalyst may also contain other metal cations such as iron, or alkaline earth cations such as magnesium and calcium and may also contain inert materials such as water and other solid dilutents. It is essential however, that the finished catalyst contain not more than 0.2 wt. % of alkali metal, and preferably, not more than 0.1 wt. %.

Suitable unprocessed aluminas are commercially available, for example, from ALCOA, Inc. under the designation H151, or H51; those sold under the trade name, "Harshaw", i.e., Harshaw Al 4028, AL 4126, and AL 4133, and those sold under the trade name "Norton", and the like. The starting alumina catalyst material should initially have a specific area of at least about 150 square meters per gram.

An important part of the invention is an improved method of alumina catalyst preparation, which activates the

alumina so that it is particularly effective in the earlier described amination reaction. To so activate the catalyst an alumina compound of the type described above is preferably first wetted in a heated steam bath for a predetermined period so as to gradually saturate the catalyst's pores with water vapor. Upon completion of this step, the catalyst particles are contacted with an acid, preferably by being immersed into an aqueous solution thereof. The alumina can be contacted with acids having either an organic or inorganic base, an acid salt or water, until the alkali metal content is reduced to below .2 wt. % and preferably, .1 wt. %. Acids which are useful in preparing such a catalyst are acetic, boric, phosphoric, hydrochloric, oxalic, citric, sulfuric and the like, with the preferred being acetic acid.

The time and temperature of the contacting should be sufficient so as to reduce the alkali metal content below the level cited above. The acid treatment is preferably followed by a water wash, although this is not a requirement for the successful performance of the catalyst.

Upon completion of the acid wash, it has been surprisingly discovered that the acidity of the alumina catalyst can be still further increased, thus making the catalyst more active, by contacting the acid washed alumina with a fluoride, or, in the broadest embodiment, an aqueous halide ion solution, wherein the catalyst will absorb halide, e.g., fluoride as, it is speculated, a substitute for oxygen in the alumina up to levels of about 2 to 15 wt. %, and preferably about 4-9 wt. %. The preferred method is to contact the alumina catalyst with an aqueous solution of the fluoride containing ion, the fluoride containing compound being selected from the group consisting of hydrofluoric acid (HF), ammonium fluoride, ammonium bifluoride, titanium tetrafluoride, and the like. The preferred fluoride containing compound is ammonium bifluoride, $NH_4FHF$, chosen for its low cost and ease in handling. Surprisingly, it has been found that there is no

substantial difference in catalyst performance as long as the final fluoride content of the catalyst is the same. Catalysts exposed to such a treatment show a definite increase in activity, and assist in substantially lowering the initial operating temperature of the process. For example, in the case of producing DPA from the reaction of aniline and phenol, the initial operating temperature can drop from about 360 - 370°C to around 330°C while the process will still exhibit substantially identical conversions and space velocities. An additional benefit is that at these lower temperatures, a significant improvement in selectivity occurs, as well as a greatly increased catalyst lifetime.

The final preferred catalyst activation treatment involves the washing and, if desired, a low temperature, e.g., 150°C, drying of the acid treated, fluorided catalyst which is now suitable for usage. Prior art treatments have usually required the calcination of the catalyst, involving first the driving off the residual wash water under controlled conditions followed by the calcining of the catalyst at 500°C for at least several hours. The improved method of this process surprisingly eliminates the need for this expensive processing step.

The following examples are provided to illustrate the invention in accordance with the principles of the invention but are not to be construed as limiting the invention in any way except as indicated by the appended claims.

## EXAMPLES

The following three examples illustrate the differences in the respective methods of catalyst manufacture.

### Example 1

No fluoriding of the catalyst.

A 1/8" Alcoa H151 alumina is initially steamed for an hour. It is then immersed in a 10% acetic acid solution for 1 hour at 90°C agitated with periodic stirring. The catalyst is then rinsed with water until obtaining a near

neutral pH, drained and calcined at 525°C for 5 hours. This procedure produces a low alkali, e.g., less than 1,000 ppm Na content, catalyst suitable for the amination of phenolics.

Example 2

A fluorided and calcined catalyst.

A 1/8" Alcoa H151 alumina is processed as in Example 1 except that, prior to calcining, the catalyst is immersed in two times its volume of 1.2 N ammonium blfluoride and held for 1 hour there in the solution kept at 80°C. The catalyst is then drained and washed repeatedly with water until the rinsings are essentially free of fluoride. The catalyst is next dried for 4 hours at 525°C. Catalyst particles prepared in this manner contain about 5.8% total fluoride content and have an alkali level of 470 ppm of Na.

Example 3

A fluorided but not calcined catalyst.

A 1/8" Alcoa H151 alumina is treated as in Example 2, except that after fluoriding, the catalyst is dried for 5 hours at 150°C instead of being calcined at 525°C.

The following three examples show the advantage of fluoriding the catalyst and that equivalent activities are obtained even when the catalyst is not heated to high temperatures (calcined).

Example 4

A 1" dia. x 14 BWG tube was charged with 200 cc of a low alkali Alcoa alumina prepared as in Example 1. A 50 wt. % aniline/50 wt. % phenol mixture was fed at a liquid hourly space velocity of 0.083 $hr^{-1}$ to a reactor operating at 800 psig. The reactor temperature required to achieve a 16% conversion of aniline and phenol to DPA was 360°C.

Example 5

Using apparatus identical to that of Example 4, 300 cc's of catalyst prepared by the method outlined in Example 2 were charged into the reactor tubes. At conditions identical to those of Example 4, a temperature of

347°C was required to achieve a 16% conversion.

Example 6

Using apparatus identical to that of Example 4, 300 cc's of a catalyst prepared by the method outlined in Example 2 were charged into the reactor tube. At conditions identical to those of Example 4, a temperature of 343°C was required to achieve a conversion of 16%.

Summarizing Examples 1-6

| Ex #'s | Catalyst Preparation | Temperature, °C for 16% Conv. at 0.083 LHSV, 800 psig |
|---|---|---|
| 1 and 4 | Acid Washed, 525°C Calcination | 360 |
| 2 and 5 | Acid Washed, fluorided, 525°C Calc. | 347 |
| 3 and 6 | Acid Washed, fluorided, 150°C Drying | 343 |

A comparison of the above data reveals the benefits of catalyst fluoridation and the non benefits resulting from high temperature calcination.

Example 7

This example illustrates the long life resulting from a catalyst prepared by the preferred method.

Using apparatus similar to that described in Example 4, 300 cc's of catalyst prepared by the method outlined in Example 3 are charged into the reactor. At conditions identical to that of Example 4, the reaction using the charged catalyst is run for 1580 hours. In that period the temperature has to be raised only 2°C to maintain a constant DPA conversion of 16%.

Examples 8-11 illustrate the benefits lower catalyst temperatures have on reaction selectivity. Lower temperatures are possible by fluoriding the alumina prior to use as outlined above.

Examples 8-11

Several amounts of fluorided catalysts were prepared using the methods outlined in Example 2 and are tested in a reactor apparatus similar to that of Example 4. The space velocities are adjusted to give 16% conversion at the reactor temperature. The following selectivity results were obtained:

were obtained:

| Example | Temp. °C | Selectivity to DPA, % |
|---|---|---|
| 8 | 330 | 95.2 |
| 9 | 345 | 93.8 |
| 10 | 353 | 92.6 |
| 11 | 390 | 90.8 |

Example 12

This example illustrates the utilization of a low alkali alumina catalyst from another alumina source. American Cyanamid alumina 'SN 5524' is a low sodium alumina, i.e. 0.027% sodium. A sample of this material was fluorided and dried using the methods of Example 3, without requiring the acetic acid treatment. 200 cc's of this material was next tested under reaction conditions described in Example 4. A temperature of 347°C was required to obtain a conversion of 16% of DPA under these conditions.

CLAIMS

1. A process for the amination of phenolic compounds comprising: contacting a phenolic based compound with an aromatic amine in a reaction zone in the presence of a solid, heterogeneous, low alkali, acidic alumina catalyst at a temperature between 300 - 450°C and at a pressure in the range from atmospheric to about 100 psia; and

withdrawing a stream containing the aminated phenolic based compound from the reaction zone.

2. A process as claimed in claim 1 wherein the phenolic based compound is phenol and the aromatic amine is aniline.

3. A process as claimed in claim 1 or claim 2 wherein the catalyst contains less than 0.2 wt. %. alkali metal.

4. A process as claimed in claim 3 wherein the catalyst contains less than 0.1 wt. %. alkali metal.

5. A process as claimed in any one of claims 1 to 3 wherein the alumina catalyst has a halide content of about 2-15 wt. %.

6. A process as claimed in claim 5 wherein the alumina catalyst has a fluoride content of about 4-9 wt. %.

7. A process as claimed in any one of claims 1 to 6 wherein the reaction temperature ranges from about 330 - 385°C.

8. A process as claimed in any one of claims 1 to 7 wherein the molar ratio of amine to phenolic compound ranges from about 0.5 to 2.0 moles of amine per mole of phenolic compound.

9. A process as claimed in any one of claims 1 to 8 wherein the process is substantially carried out in the liquid phase.

10. A process as claimed in any one of claims 1 to 9 wherein the process is a batch process.

11. A process as claimed in any one of claims 1 to 9 wherein the process has a liquid hourly space velocity ranging from about 0.01 to 0.50 $hr^{-1}$.

12. A process for the preparation of an acidic alumina catalyst having a low alkali metal content, without involving a high temperature calcination treatment, comprising; contacting a solid, heterogeneous alumina with an effective acid of organic or inorganic base, an acid salt or water, for a sufficient time to reduce the alumina alkali metal content to a predetermined low level;

further contacting the alumina with an aqueous halide ion containing solution for a sufficient time so as to absorb about 2-15 wt. %. of the halide ion into the alumina;

washing the treated catalyst and drying it in a low temperature environment.

13. A process as claimed in claim 12 wherein the acid used to reduce the alkali metal content of the alumina is selected from acetic, boric, phosphoric, hydrochloric, oxalic, citric, and sulfuric acids.

14. A process as claimed in claim 12 or claim 13 wherein the alumina alkali metal content is reduced to less than 0.2 wt. %.

15. A process as claimed in any one of claims 12 to 14 where the halide ion containing solution contains fluoride ion.

16. A process as claimed in claim 15 wherein the fluoride ion containing compound is selected from hydrofluoric acid, ammonium fluoride, ammonium bifluoride and titanium tetrafluoride.

17. A process as claimed in claim 15 or claim 16 where about 4-9 wt. %. of fluoride is absorbed into the alumina catalyst.

18. A process as claimed in any one of claims 12 to 17 wherein the acidified and halide ion treated catalyst is dried at temperatures of about 150°C.

19. A process for the activation of a solid, heterogeneous, acidic, halide containing alumina catalyst without involving a high temperature calcination treatment comprising; contacting a crystalline alumina with acetic acid for time sufficient to reduce the alumina alkali metal content to below a predetermined low level;

further contacting the alumina with an aqueous solution of ammonium bifluoride so that the alumina absorbs about 4-9 wt. %. of the fluoride;

washing the treated alumina and drying it at a low temperature.

20. A process as claimed in any one of claims 1 to 11 wherein the catalyst has been prepared by a process as claimed in any one of claims 12 to 19.